# EUROPEAN PATENT SPECIFICATION

(11) **EP 1 405 619 B1**
(45) Date of publication and mention of the grant of the patent: **04.11.2009**
(21) Application number: 03256162.3
(22) Date of filing: 30.09.2003
(51) Int. Cl.: A61F 13/535, A61F 13/539, A61F 13/47, A61F 13/49

(54) **Urine guiding article and use of the same**
Artikel zur Führung von Urin und Verwendung dafür
Article pour le guidage de l'urine et son utilisation

(30) Priority: 30.09.2002 JP 2002287462
(43) Date of publication of application: 07.04.2004
(73) Proprietor: UNI-CHARM CORPORATION, Shikokuchuo-shi, Ehime-ken (JP)
(72) Inventor: Kawata, Hikari, c/o Technical Center, Mitoyo-gun, Kagawa-ken 769-1602 (JP); Wada, Ichiro, c/o Technical Center, Mitoyo-gun, Kagawa-ken 769-1602 (JP)
(74) Representative: Eke, Philippa Dianne

(56) References cited:
- EP-A- 0 933 074
- US-A- 4 022 210
- US-A- 4 735 624
- US-A- 5 392 467
- US-A- 5 451 442
- US-B1- 6 344 036

## Description

The present invention relates to urine guiding articles and the same used in combination with a body fluid absorbent wearing article such as disposable diapers or urine-absorbent pads to guide a discharged urine flow downward to such body fluid absorbent wearing article after urination.

It is known, for example, from Japanese Utility Model Application No. 1994-48638A to form an absorbent pad of a disposable diaper with compressed grooves adapted to guide a urine flow downward. Such compressed grooves are effective to prevent body fluids such as urine from leaking sideways beyond the peripheral edge of the diaper.

However, such compressed grooves are previously formed in the disposable diaper and it will be obvious that the effect offered by the compressed grooves can not be utilized if the user purchases the diaper formed with none of these compressed grooves.

Further prior art absorbent articles are known from US4735624, which discloses an absorbent article comprising an absorbent material consisting of hydrophilic fibres joined together to form a coherent structure having at least three branches that diverge from one another; US 5451442, which discloses an absorbent panel that features elongated areas or grooves of reduced thickness; EP 0933074, which discloses an absorbent article featuring a core that is divided along longitudinal lines; and US 6344036, which discloses a material layer provided with a plurality of laterally arranged slit formations.

It is an object of the present invention to provide a simplified means adapted to guide a discharged urine flow in a body fluid absorbent wearing article without relying upon compressed grooves formed in such body fluid absorbent wearing article.

According to an aspect of the present invention, there is provided a urine guiding article and, according to another aspect of the present invention, there is provided use of such urine guiding article.

The present invention includes a urine guiding article having a longitudinal direction and a transverse direction, said urine guiding article further comprising: a plurality of ribbon-like strips extending along said longitudinal direction while being arranged in said transverse direction and having proximal end portions and distal end portions; and a sheet-like connecting region adapted to interconnect said proximal end portions of said plurality of ribbon-like strips, whereby an amount of urine discharged on said plurality of ribbon-like strips is guided therealong from said proximal end portions toward said distal end portions, characterised in that each of said ribbon-like strips comprises a fibrous assembly in which component fibers are oriented in said longitudinal direction.

The ribbon-like strips are adapted to spread the amount of urine in the longitudinal direction rather than in the transverse direction.

The ribbon-like strips comprise hydrophobic fibers.

The ribbon-like strips comprise, in addition to the hydrophobic fibers, hydrophilic fibers also.

The connecting region is coated on one surface thereof with an adhesive.

The adhesive is one for permanently attaching an inner surface of a body fluid absorbent wearing article.

The adhesive is one for detachably attaching an inner surface of a body fluid absorbent wearing article.

According to another aspect of this invention, the urine guiding article is used in combination with a body fluid absorbent wearing article in a manner that the connecting region of the urine guiding article is attached to an inner surface of the body fluid absorbent wearing article.
Fig. 1 is a perspective view showing a urine guiding article;
Fig. 2 is a diagram illustrating the urine guiding article as put on the wearer's body; and
Fig. 3 is a partially cutaway perspective view illustrating how the urine guiding article is used in a manner different from the manner shown in Fig. 2.

Details of the urine guiding article according to the present invention will be more fully understood from the description given hereunder with reference to the accompanying drawings.

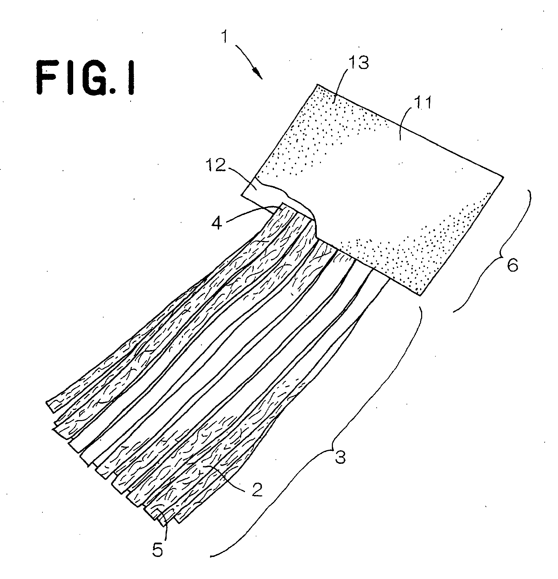

A urine guiding article 1 illustrated in Fig. 1 comprises a urine guiding region 3 including a plurality of ribbon-like strips 2 extending parallel one to another obliquely downward as viewed in Fig. 1 and a sheet-like connecting region 6 extending in a transverse direction and interconnecting upper end portions of the individual ribbon-like strips 2 one with another.

Each of the ribbon-like strips 2 preferably has a width of 3 mm to 30 mm and a length of 50 mm to 400 mm. A sheet material for such ribbon-like strip 2 may be selected from the group including a nonwoven fabric, a woven fabric, a film and a paper. When a nonwoven fabric or a woven fabric is used as this sheet material, such nonwoven or woven fabric may be constituted by hydrophobic fibers, hydrophilic fibers or a mixture of hydrophobic fibers and hydrophilic fibers. The fiber is oriented in a longitudinal direction of the ribbon-like strip 2.

similarly to the ribbon-like strips 2, a stock material for the connecting region 6 may be selected from the group including a nonwoven fabric, a woven fabric, a film and a paper. The respective upper end portions 4 of the ribbon-like strips 2 are secured selectively by various means such as adhesion, heat-welding and stitching. In the illustrated embodiment, the connecting region 6 includes an inner sheet 11 coated with a self-adhesive 13 and an outer sheet 12 laid on the backside of the inner sheet 11. As illustrated, a plurality of the ribbon-like strips 2 are interposed between the inner and outer sheets 11, 12 constituting the connecting region 6 are arranged side by side in the transverse direction so as to overlap themselves two by two or three by three.

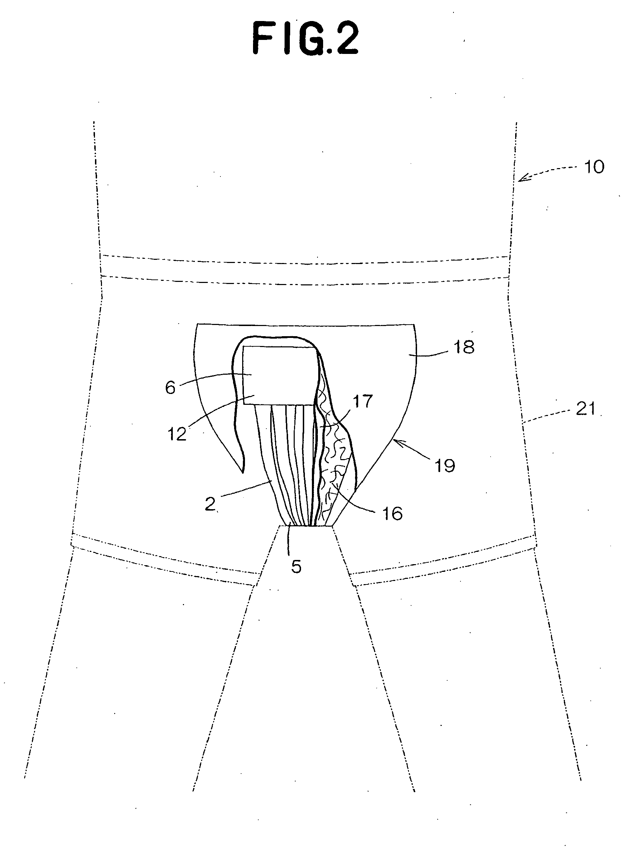

Fig. 2 shows the urine guiding article 1 as put on a wearer's body. The inner sheet 11 of the urine guiding article 1 is releasably attached to a belly of the wearer 10 by means of the self-adhesive 13 applied on the inner sheet 11. The ribbon-like strips 2 extend downward from the belly of the wearer 10 toward a crotch region of the wearer 10 so as to gather together and consequently have a total width gradually reduced and cover urethral (not shown). On the outer side of the urine guiding article 1, a conventional urine-absorbent pad 19 comprising a liquid-pervious topsheet 17, a liquid-impervious backsheet 18 and a liquid-absorbent core 16 interposed between these two sheets 17, 18 is placed. On the outer side of the urine-absorbent pad 19, briefs 21 are placed to maintain the pad 19 in close contact with the crotch region of the wearer 10 as indicated by imaginary lines. By using the urine guiding article 1 in this manner, it is ensured that an amount of urine discharged onto a portion of the ribbon-like strips 2 facing the urethral wet this portion and flow downward to lower end portions 5 of the ribbon-like strips 2, in other words, to a crotch region of the urine-absorbent pad 19 rather than to transversely side edges of the urine-absorbent pad 19. In this way, use of the urine guiding article 1 makes it possible to prevent any amount of urine from leaking sideways in the urine-absorbent pad 19.

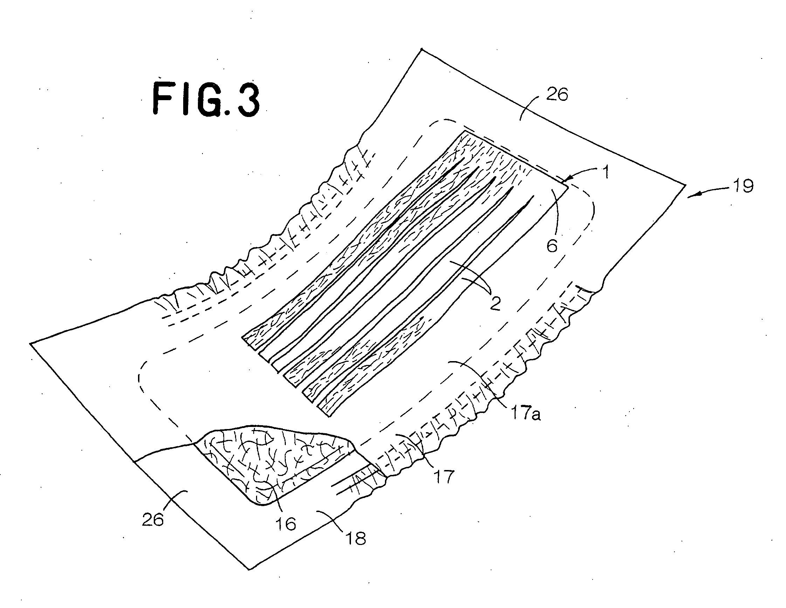

Fig. 3 is a partially cutaway perspective view showing the urine-absorbent pad 19 to illustrate an example of how the urine guiding article 1 is used. The urine-absorbent pad 19 comprises, similarly to that illustrated in Fig. 2, the liquid-pervious topsheet 17, the liquid-impervious backsheet 18 and the body fluid absorbent core 16. The connecting region 6 of the urine guiding article 1 is attached to the inner surface 17a of the topsheet 17 by various means such as adhesion, pressure sensitive adhesion and welding. Of the urine guiding article 1, the connecting region 6 lies in the vicinity of one of longitudinally opposite ends 26 of the urine-absorbent pad 19 and the ribbon-like strips 2 extend between the longitudinally opposite ends 26. Use of the urine-absorbent pad 19 arranged in this manner makes it unnecessary to attach the urine guiding article 1 to the wearer's body like the urine guiding article 1 illustrated in Fig. 2. The urine-absorbent pad 19 illustrated in Fig. 3 can be made by the wearer him- or herself as long as a conventional urine-absorbent pad and the urine guiding article 1 illustrated in Fig. 1 are available for the wearer. In the illustrated embodiment of the urine guiding article 1, the ribbon-like strips 2 and the connecting region 6 are made of the same material and a plurality of the ribbon-like strips 2 are arranged side by side in the transverse direction

While the urine guiding article 1 has been described in combination with the urine-absorbent pad 19, this urine guiding article 1 may be used in combination with the conventional disposable diaper without departing from the present invention. Of the urine guiding article 1, the ribbon-like strips 2 are preferably configured so as to spread a discharged amount of urine more rapidly in the longitudinal direction than in the transverse direction. The ribbon-like strips 2 offering such desired effect are obtained by orienting the component fibers of the respective strips 2 in the longitudinal direction of the strips 2. The component fibers which are hydrophobic and oriented in the longitudinal direction facilitate the urine flow to be guided toward the lower end portions 5 of the respective strips 2. These strips 2 are distinguished from the compressed grooves of the core as has been described at the beginning, the orientation of the component fibers forming the respective strips 2 may be selected within a relatively wide range to determine a direction in which the discharged amount of urine is desired to be guided by these strips 2.

The urine guiding article according to the present invention may be releasably attached to the wearer's body or to the inner surface of the urine-absorbent pad or the disposable diaper to guide the urine flow in the direction in which the respective strips of the urine guiding article extend. By use of this urine guiding article, it is possible to prevent any amount of urine from leaking sideways in the disposable diaper or the like and it becomes no more necessary to form the body fluid absorbent core with the compressed grooves.

## Claims

1. A urine guiding article (1) having a longitudinal direction and a transverse direction, said urine guiding article further comprising:
a plurality of ribbon-like strips (2) extending along said longitudinal direction while being arranged in said transverse direction and having proximal end portions (4) and distal end portions (5); and
a sheet-like connecting region (6) adapted to interconnect said proximal end portions of said plurality of ribbon-like strips,
whereby an amount of urine discharged on said plurality of ribbon-like strips is guided therealong from said proximal end portions toward said distal end portions,
**characterised in that** each of said ribbon-like strips comprises a fibrous assembly in which component fibers are oriented in said longitudinal direction.

2. The article according to Claim 1, wherein said ribbon-like strips are adapted to spread an amount of urine in said longitudinal direction rather than in said transverse direction.

3. The article according to Claim 1, wherein said ribbon-like strips comprise hydrophobic fibers.

4. The article according to Claim 1, wherein said ribbon-like strips comprise, in addition to said hydrophobic fibers, hydrophilic fibers also.

5. The article according to Claim 1, wherein said connecting region is coated on one surface thereof with an adhesive

6. The article according to Claim 5, wherein said adhesive is one for permanently attaching said article to an inner surface of a body fluid absorbent wearing article.

7. The article according to Claim 5, wherein said adhesive is one for detachably attaching said article to one of an inner surface of a body fluid absorbent wearing article and the body of a wearer.

8. The use of said urine guiding article according to Claim 1 in combination with a body fluid absorbent wearing article wherein said connecting region is attached to an inner surface of said body fluid absorbent wearing article.

## Patentansprüche

1. Urinführungsartikel (1) mit einer Längs- und einer Querrichtung, besagter Urinführungsartikel weiter umfassend:
eine Vielzahl von bandartigen Streifen (2), die in besagter Längsrichtung verlaufen und in besagter Querrichtung angeordnet sind und die proximale Endabschnitte (4) und distale Endabschnitte (5) aufweisen; und
einen tuchartigen Verbindungsbereich (6) zum Verbinden der besagten proximalen Endabschnitte der besagten Vielzahl von bandartigen Streifen,
wobei eine auf besagte Vielzahl von bandartigen Streifen abgelassene Urinmenge an diesen entlang von besagten proximalen Endabschnitten zu besagten distalen Endabschnitten geführt wird,
**dadurch gekennzeichnet, dass** die besagten bandartigen Streifen jeweils eine faserige Baueinheit aufweisen, deren Faserbestandteile in besagter Längsrichtung ausgerichtet sind.

2. Artikel nach Anspruch 1, wobei die besagten bandartigen Streifen so beschaffen sind, dass sie eine Urinmenge nicht in besagter Querrichtung, sondern in besagter Längsrichtung verbreiten.

3. Artikel nach Anspruch 1, wobei die besagten bandartigen Streifen hydrophobe Fasern beinhalten.

4. Artikel nach Anspruch 1, wobei die besagten bandartigen Streifen zusätzlich zu besagten hydrophoben Fasern auch hydrophile Fasern beinhalten.

5. Artikel nach Anspruch 1, wobei der besagte Verbindungsbereich auf einer seiner Oberflächen mit einem Klebstoff beschichtet ist.

6. Artikel nach Anspruch 5, wobei es sich bei besagtem Klebstoff um einen handelt, der den besagten Artikel bleibend an einer Innenfläche eines Körperflüssigkeit absorbierenden Bekleidungsartikels befestigt.

7. Artikel nach Anspruch 5, wobei es sich bei besagtem Klebstoff um einen handelt, der den besagten Artikel abnehmbar an einer Innenfläche eines Körperflüssigkeit absorbierenden Bekleidungsartikels und am Körper eines den Artikel tragenden Menschen befestigt.

8. Anwendung des besagten Urinführungsartikels nach Anspruch 1 in Verbindung mit einem Körperflüssigkeit absorbierenden Bekleidungsartikel, wobei der besagte Verbindungsbereich an einer Innenfläche des besagten Körperflüssigkeit absorbierenden Bekleidungsartikels befestigt ist.

## Revendications

1. Article (1) pour le guidage de l'urine ayant un sens longitudinal et un sens transversal, ledit article pour le guidage de l'urine comportant par ailleurs :
une pluralité de bandes similaires à des rubans (2) s'étendant le long dudit sens longitudinal tout en étant arrangées dans ledit sens transversal et ayant des portions extrêmes proximales (4) et des portions extrêmes distales (5) ; et
une région de raccordement similaire à une feuille (6) adaptée pour raccorder ensemble lesdites portions extrêmes proximales de ladite pluralité de bandes similaires à des rubans,
ce par quoi une quantité d'urine déchargée sur ladite pluralité de bandes similaires à des rubans est guidée le long de celles-ci depuis lesdites portions extrêmes proximales vers lesdites portions extrêmes distales,
**caractérisé en ce que** chacune desdites bandes similaires à des rubans comporte un ensemble fibreux dans lequel des fibres constitutives sont orientées dans ledit sens longitudinal.

2. Article selon la revendication 1, dans lequel lesdites bandes similaires à des rubans sont adaptées pour étaler une quantité d'urine dans ledit sens longitudinal plutôt que dans ledit sens transversal.

3. Article selon la revendication 1, dans lequel lesdites bandes similaires à des rubans comportent des libres hydrophobes.

4. Article selon la revendication 1, dans lequel lesdites bandes similaires à des rubans comportent également, en plus desdites fibres hydrophobes, des fibres hydrophiles.

5. Article selon la revendication 1, dans lequel ladite région de raccordement est revêtue d'un adhésif sur une surface de celle-ci.

6. Article selon la revendication 5, dans lequel ledit adhésif est un adhésif destiné à attacher de manière permanente ledit article sur une surface intérieure d'un vêtement absorbant des liquides corporels.

7. Article selon la revendication 5, dans lequel ledit adhésif est un adhésif destiné à attacher de manière détachable ledit article sur l'un quelconque d'une surface intérieure d'un vêtement absorbant des liquides corporels et du corps d'un utilisateur.

8. Utilisation dudit article pour le guidage de l'urine selon la revendication 1 en combinaison avec un vêtement absorbant des liquides corporels, dans lequel ladite région de raccordement est attachée à une surface intérieure dudit vêtement absorbant des liquides corporels.
